# EUROPÄISCHE PATENTSCHRIFT

(11) **EP 3 459 586 B1**
(45) Veröffentlichungstag und Bekanntmachung des Hinweises auf die Patenterteilung: **15.01.2025**
(21) Anmeldenummer: 18190373.3
(22) Anmeldetag: 23.08.2018
(51) Int. Cl.: A61M 39/10

(54) **VERBINDUNGSSYSTEM FÜR EIN MEDIZINISCHES FLUIDLEITUNGSSYSTEM SOWIE VERBINDUNGS- UND ANSCHLUSSEINHEIT FÜR EIN SOLCHES VERBINDUNGSSYSTEM**
CONNECTION SYSTEM FOR A MEDICAL FLUID PIPING SYSTEM AND CONNECTING AND TERMINAL UNIT FOR SUCH A CONNECTION SYSTEM
SYSTÈME DE RACCORD POUR UN SYSTÈME DE CONDUITES DE FLUIDE MÉDICAL AINSI QU'UNITÉ DE RACCORD ET DE CONNEXION POUR UN TEL SYSTÈME DE RACCORD

(30) Priorität: 26.09.2017 DE 102017217092
(43) Veröffentlichungstag der Anmeldung: 27.03.2019
(73) Patentinhaber: B. Braun Melsungen AG, 34212 Melsungen (DE)
(72) Erfinder: Fuchs, Jürgen, 34308 Bad Emstal (DE)
(74) Vertreter: Patentanwälte Ruff, Wilhelm, Beier, Dauster & Partner mbB

(56) Entgegenhaltungen:
- EP-A2- 0 114 677
- WO-A1-2010/071812
- WO-A1-2016/007166
- WO-A1-2017/091635
- DE-A1- 10 321 309

## Beschreibung

Die Erfindung betrifft ein Verbindungssystem für ein medizinisches Fluidleitungssystem aufweisend eine Verbindungseinheit mit einem Verbindungskörper, der einen distal angeordneten Luer-Außenkegel und einen koaxial zu dem Luer-Außenkegel erstreckten Durchgangskanal aufweist, und eine Anschlusseinheit mit einem Anschlusskörper, der einen proximal angeordneten Luer-Innenkegel, der lösbar fluiddicht mit dem Luer-Außenkegel der Verbindungseinheit verbunden ist, ein koaxial zu dem Luer-Innenkegel sowie proximal angeordnetes Außengewinde, das zur Verbindung mit einem Innengewinde eines Luer-Lock-Ansatzes geeignet ist, und ein koaxial zu dem Luer-Innenkegel erstrecktes und mittels der Verbindung der Luer-Kegel lösbar fluidleitend mit dem Durchgangskanal verbundenes Lumen aufweist, sowie eine Verbindungs- und eine Anschlusseinheit, die jeweils nach den vorstehenden Ausführungen gestaltet sind.

Ein derartiges Verbindungssystem mit einer Verbindungs- sowie einer Anschlusseinheit ist im Bereich der Medizintechnik allgemein bekannt, zum Beispiel aus der WO 2010/071812 A1. Die bekannte Verbindungseinheit ist
beispielsweise in Form eines Infusionsschlauchverbinders und die bekannte Anschlusseinheit beispielsweise in Form eines Venenverweilkatheters ausgebildet. Der Infusionsschlauchverbinder weist einen Verbindungskörper in Form eines Schlauchansatzes mit einem Durchgangskanal auf. Distal an dem Verbindungskörper ist ein Luer-Außenkegel, proximal ein Befestigungsabschnitt zur Verbindung mit einem Infusionsschlauch vorgesehen. Der Venenverweilkatheter weist einen Anschlusskörper in Form eines Katheteransatzes mit einem durchgehend erstreckten Lumen und einen Luer-Innenkegel auf. Die Luer-Kegel sind auf eine im Bereich der Medizintechnik grundsätzlich bekannte Weise fluiddicht miteinander verbindbar, so dass eine durchgängige fluidleitende Verbindung ausgehend von dem Infusionsschlauch über den Durchgangskanal des Schlauchansatzes in das Lumen des Katheteransatzes erreicht wird. Bei dem bekannten Verbindungssystem ist zur Sicherung der Luer-Kegel-Verbindung eine Schraubverbindung zwischen einem Außengewinde des Katheteransatzes und einem Innengewinde des Schlauchansatzes vorgesehen, die auch als Luer-Lock-Verbindung bezeichnet wird. Eine solche Luer-Lock-Verbindung schließt nicht in allen Fällen zuverlässig. Zum einen kann es beispielsweise aufgrund von Fertigungstoleranzen oder einem zu leichten Anziehen der Schraubverbindung zu einem spontanen ungewollten Lösen der Luer-Kegel-Verbindung kommen. Zum anderen kann eine zu fest angezogene Schraubverbindung nicht mehr manuell, sondern lediglich unter Zuhilfenahme zusätzlicher Werkzeuge zu lösen sein. In beiden Fällen kann die Patientensicherheit beeinträchtigt werden.

Aufgabe der Erfindung ist es, ein Verbindungssystem, eine Verbindungseinheit sowie eine Anschlusseinheit der eingangs genannten Art zu schaffen, die gegenüber dem Stand der Technik verbesserte Eigenschaften aufweisen und insbesondere eine zu einer Luer-Lock-Verbindung bedarfsweise alternative Art der Sicherung der Luer-Kegel-Verbindung ermöglichen.

Diese Aufgabe wird für ein Verbindungssystem der eingangs genannten Art dadurch gelöst, dass eine lösbare Rastverbindung zwischen wenigstens einem an dem Verbindungskörper angeordneten Rastelement und wenigstens einem an dem Anschlusskörper angeordneten komplementären Gegenrastelement vorgesehen ist, wobei die Rastverbindung derart gestaltet ist, dass einem unbeabsichtigten Lösen der Verbindung der Luer-Kegel entgegengewirkt ist, und wobei das Gegenrastelement und das Außengewinde derart gestaltet und angeordnet sind, dass die Anschlusseinheit - in einem von der Verbindungseinheit getrennten Zustand - mittels einer Schraubverbindung zwischen dem Außengewinde und dem Innengewinde des Luer-Lock-Ansatzes verbindbar ist. Die erfindungsgemäße Lösung ermöglicht zum einen - mittels der vorgesehenen Rastverbindung - ein zuverlässiges Sichern und bei Bedarf leichtes Lösen der Luer-Kegel-Verbindung zwischen der Anschluss- und der Verbindungseinheit. Zum anderen wird erfindungsgemäß durch das an der Anschlusseinheit vorgesehene Außengewinde erreicht, dass die Anschlusseinheit im Bedarfsfall stattdessen mit einem Luer-Lock-Ansatz, beispielsweise in Form einer Verschlusskappe oder dergleichen, verschraubbar ist. Die erfindungsgemäße Lösung sieht hierzu eine geeignete Ausgestaltung und/oder Anordnung des Außengewindes und des Gegenrastelements vor. Hierbei ist das Außengewinde derart gestaltet, dass eine Verbindung mit einem normgerechten Luer-Lock-Ansatz und zudem - sofern statt des Luer-Lock-Ansatzes eine erfindungsgemäße Verbindungseinheit mit der Anschlusseinheit verbunden werden soll - eine Verbindung des Rast- mit dem Gegenrastelement gewährleistet ist, ohne dass das Außengewinde hierbei die Rastverbindung in Form eines Hindernisses unterbindet, erschwert oder blockiert. Das Außengewinde kann zum Zweck der erfindungsgemäßen Gestaltung beispielsweise - ausgehend von einer standardmäßigen Luer-Lock-Gewindegeometrie - mit wenigstens einer radial und/oder axial erstreckten Abflachung, Ausnehmung, Lücke oder dergleichen versehen sein. Das Gegenrastelement kann insbesondere durch einen Abschnitt des Außengewindes gebildet oder als eine gesonderte Rastgeometrie an dem Anschlusskörper vorgesehen sein. Das wenigstens eine Rastelement kann beispielsweise in Form eines Rasthakens, einer Rastnase oder dergleichen ausgebildet sein. Dementsprechend komplementär kann das Gegenrastelement beispielsweise in Form einer Rastöffnung, einer Nut oder dergleichen ausgebildet sein. Es ist auch möglich, dass das Rast- und das Gegenrastelement umgekehrt komplementär zu den vorstehenden Ausführungen ausgebildet sind.

Die der Erfindung zugrunde liegende Aufgabe wird für eine Verbindungseinheit der eingangs genannten Art dadurch gelöst, dass der Verbindungskörper ein Rastelement und einen dem Rastelement zugeordneten Handhabungsabschnitt aufweist, wobei das Rastelement und der Handhabungsabschnitt derart angeordnet und/oder gestaltet sind, dass das Rastelement mittels einer manuellen Handhabung des Handhabungsabschnitts - in Bezug auf einen mit der Anschlusseinheit verbundenen Zustand - relativ zu dem Luer-Außenkegel von einer Rastposition, in der das Rastelement an einem Gegenrastelement der Anschlusseinheit verrastet ist, in eine Freigabeposition, in der das Rastelement das Gegenrastelement freigibt, verlagerbar ist. Die erfindungsgemäße Lösung ermöglicht demnach ein manuell leichtgängiges Lösen und Verbinden der Rastverbindung. Das Rastelement kann kraft-, form- und/oder stoffschlüssig mit dem Handhabungsabschnitt verbunden sein. Der Handhabungsabschnitt kann vorzugsweise in Form einer Betätigungsplatte, eines Schiebers, eines Riegels oder dergleichen an dem Verbindungskörper ausgebildet sein. Das Rastelement kann mittels einer Druck-, Zug- und/oder Schubbetätigung des Handhabungsabschnitts von der Rastposition in die Freigabeposition verlagerbar sein. Die Verlagerung des Rastelements kann hierbei relativ zu dem Luer-Außenkegel in axialer, radialer und/oder tangentialer Richtung erfolgen. Die Verbindungseinheit kann insbesondere in Form eines Infusionsschlauchverbinders oder einer Spritze ausgestaltet sein. Die erfindungsgemäße Lösung ist jedoch nicht auf eine derartige Ausgestaltung der Verbindungseinheit beschränkt, sondern auch für andere Verbindungseinheiten für medizinische Fluidleitungssysteme geeignet.

In Ausgestaltung der Erfindung ist das Rastelement mittels einer - in Bezug auf eine Mittellängsachse der Verbindungseinheit - radial nach innen gerichteten elastischen Verlagerung des Handhabungsabschnitts radial nach außen elastisch in die Freigabeposition verlagerbar. Der Handhabungsabschnitt kann als solcher elastisch ausgestaltet oder stattdessen elastisch verlagerbar an dem Verbindungskörper, beispielsweise mittels eines Federelements, abgestützt sein. Dementsprechend kann das Rastelement elastisch nachgiebig gestaltet oder elastisch an dem Verbindungskörper abgestützt sein. Die radial nach innen gerichtete Verlagerung des Handhabungsabschnitts ist vorzugsweise mittels einer manuellen Druckbetätigung bewirkbar.

In weiterer Ausgestaltung der Erfindung weist der Verbindungskörper einen biegeelastischen Gelenkabschnitt auf, an dem das Rastelement in distaler Richtung und der Handhabungsabschnitt in proximaler Richtung erstreckt und in einer Mittellängsebene der Verbindungseinheit verschwenkbar angelenkt sind. Der Gelenkabschnitt ist vorzugsweise in Form eines im Wesentlichen radial zu dem Luer-Außenkegel erstreckten Wandabschnittes gefertigt. Der Gelenkabschnitt ist derart angeordnet und ausgestaltet, dass eine Druckbelastung des Handhabungsabschnitts eine Biegedeformation des Gelenkabschnitts in der Mittellängsebene bewirkt, wobei der Handhabungsabschnitt gegenüber dem Luer-Außenkegel radial nach innen und das Rastelement radial nach außen verschwenkt werden.

In weiterer Ausgestaltung der Erfindung sind mehrere, vorzugsweise zwei, in Umfangsrichtung gleichmäßig versetzt zueinander angeordnete Rastelemente mit jeweils einem zugeordneten Handhabungsabschnitt sowie einem Gelenkabschnitt vorgesehen. Soweit zwei in Umfangsrichtung um 180° versetzt zueinander angeordnete Rastelemente vorgesehen sind, ergibt sich eine besonders ergonomische Handhabbarkeit. In einem solchen Fall kann die Rastverbindung mittels einer Betätigung der gegenüberliegenden Handhabungsabschnitte zwischen beispielsweise Daumen und Zeigefinger erfolgen.

In weiterer Ausgestaltung der Erfindung weist der Verbindungskörper wenigstens einen in axialer Richtung erstreckten Führungssteg auf, der zur axialen Führung in einer komplementären Führungsnut der Anschlusseinheit vorgesehen ist. Diese Ausgestaltung der Erfindung erlaubt eine vereinfachte Ausrichtung der Verbindungseinheit gegenüber der Anschlusseinheit bei einem Herstellen der Rastverbindung zwischen dem Rastelement und dem Gegenrastelement.

In weiterer Ausgestaltung der Erfindung sind mehrere, vorzugsweise zwei, in Umfangsrichtung gleichmäßig versetzt zueinander angeordnete Führungsstege vorgesehen. Sofern die Führungsstege geometrisch unterschiedlich zueinander ausgeführt sind, kann vorteilhafterweise eine in Umfangsrichtung bestimmte Ausrichtung der Verbindungseinheit gegenüber der Anschlusseinheit definiert werden.

In weiterer Ausgestaltung der Erfindung ist ein stirnendseitig an den Luer-Außenkonus angespritztes Dichtelement vorgesehen. Das Dichtelement ist vorzugsweise gummielastisch ausgeführt. Durch diese Ausgestaltung der Erfindung kann eine verbesserte Dichtigkeit der Verbindung der Luer-Kegel erreicht werden.

Die der Erfindung zugrunde liegende Aufgabe wird für eine Anschlusseinheit der eingangs genannten Art dadurch gelöst, dass der Anschlusskörper ein Gegenrastelement zur lösbaren Rastverbindung mit einem Rastelement der Verbindungseinheit aufweist, und wobei das Gegenrastelement und das Außengewinde derart angeordnet und gestaltet sind, dass die Anschlusseinheit mit der Verbindungseinheit mittels einer Rastverbindung zwischen dem Gegenrastelement und dem Rastelement und - stattdessen - mittels einer Schraubverbindung zwischen dem Außengewinde und einem Innengewinde des Luer-Lock-Ansatzes verbindbar ist.

Demzufolge ermöglicht die erfindungsgemäße Lösung zum einen ein zuverlässiges Sichern der Luer-Kegel-Verbindung zwischen der Anschluss- und der Verbindungseinheit mittels der lösbaren Rastverbindung zwischen dem Rast- und dem Gegenrastelement. Zum anderen wird erfindungsgemäß erreicht, dass die Anschlusseinheit - anstelle mit einer erfindungsgemäßen Verbindungseinheit - mit einem normgerechten Luer-Lock-Ansatz verschraubbar ist. Zu diesem Zweck sieht die erfindungsgemäße Lösung eine geeignete Ausgestaltung und/oder Anordnung des Außengewindes und des Gegenrastelements vor. Das Außengewinde ist hierbei derart gestaltet, dass insbesondere eine Verbindung des Gegenrastelements mit dem Rastelement gewährleistet ist, ohne dass das Außengewinde hierbei die Rastverbindung in Form eines Hindernisses unterbindet, erschwert oder blockiert. Entsprechend ist das Gegenrastelement insbesondere derart gestaltet, dass eine Schraubverbindung mit dem Außengewinde nicht blockiert oder erschwert ist. Das Außengewinde kann zum Zwecke der erfindungsgemäßen Gestaltung beispielsweise - ausgehend von einer standardmäßigen Luer-Lock-Gewindegeometrie - mit einer radialen und/oder axial erstreckten Abflachung, Ausnehmung, Lücke oder dergleichen versehen sein.

Erfindungsgemäß grenzt das Gegenrastelement an einen distalen Stirnendbereich des Außengewindes an und ist in radialer Richtung gegenüber einem Durchmesser, vorzugsweise einem Kerndurchmesser, des Außengewindes zurückversetzt. Demzufolge ist das Gegenrastelement - in einem mit der Verbindungseinheit verbundenen Zustand der Anschlusseinheit - an einer der Verbindungseinheit abgewandten Seite des Außengewindes an dem Anschlusskörper angeordnet. Dies ermöglicht insbesondere ein durch das Gegenrastelement ungehindertes Aufschrauben eines Luer-Lock-Ansatzes auf das Außengewinde. Durch die radial gegenüber dem Außengewinde zurückversetzte Anordnung des Gegenrastelements wird eine Behinderung des Außengewindes bei einem Auf- oder Abschrauben des Luer-Lock-Ansatzes vermieden.

In weiterer Ausgestaltung der Erfindung ist das Gegenrastelement in Form einer in Umfangsrichtung, vorzugsweise vollständig umlaufend, erstreckten Radialnut an dem Anschlusskörper ausgebildet. In einem mit der Verbindungseinheit verbundenen Zustand greift das Rastelement formschlüssig in die Radialnut ein. Hierdurch ist einem ungewollten Lösen der Luer-Kegel-Verbindung bzw. einem ungewollten Abziehen der Verbindungseinheit von der Anschlusseinheit oder umgekehrt entgegengewirkt.

In weiterer Ausgestaltung der Erfindung ist das Außengewinde in Axialrichtung von wenigstens einer Führungsnut, die zur Führung eines Führungsstegs der Verbindungseinheit vorgesehen ist, durchsetzt. Das Außengewinde ist somit in Umfangsrichtung abschnittsweise unterbrochen.

Demzufolge gewährleistet die Führungsnut zum einen eine lagerichtige Ausrichtung der Anschluss- gegenüber der Verbindungseinheit und somit eine verbesserte Handhabung. Zum anderen wird durch die formschlüssige Führung zwischen der Führungsnut und dem Führungssteg eine Verdrehsicherung zwischen der Anschluss- und der Verbindungseinheit erreicht.

In weiterer Ausgestaltung der Erfindung weist das Außengewinde wenigstens einen gegenüber einem Außendurchmesser des Außengewindes radial abgeflachten Gewindeabschnitt auf, der in einem mit der Verbindungseinheit verbundenen Zustand der Anschlusseinheit von dem Rastelement hintergriffen ist. Die Abmessungen, insbesondere das Maß der radialen Abflachung, des Gewindeabschnitts sind auf die Abmessungen des Rastelements abgestimmt. Auf diese Weise ist gewährleistet, dass das Rastelement bei einer Verbindung der Anschlussmit der Verbindungseinheit in axialer Richtung über den abgeflachten Gewindeabschnitt hinweggeführt werden kann. In einem an dem Gegenrastelement verrasteten Zustand hintergreift das Rastelement den abgeflachten Gewindeabschnitt.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in einer schematischen Perspektivdarstellung eine Ausführungsform eines erfindungsgemäßen Verbindungssystems, das eine Ausführungsform einer erfindungsgemäßen Verbindungseinheit sowie eine Ausführungsform einer erfindungsgemäßen Anschlusseinheit aufweist,
- Fig. 2: in einer schematischen Perspektivdarstellung die Anschlusseinheit nach Fig. 1, wobei anstelle der Verbindungseinheit nach Fig. 1 eine Verbindungseinheit nach dem Stand der Technik mit einem standardmäßigen Luer-Lock-Ansatz mit der Anschlusseinheit verbunden ist,
- Fig. 3: in einem schematischen Längsschnitt in einer Mittellängsebene die Verbindungseinheit nach Fig. 1,
- Fig. 4: in einer schematischen Perspektivdarstellung in Blickrichtung auf ein distales Ende die Verbindungseinheit nach den Fig. 1 und 3,
- Fig. 5: in einer schematischen Perspektivdarstellung die Anschlusseinheit nach den Fig. 1 und 2,
- Fig. 6: in einer vergrößerten schematischen Perspektivdarstellung die Anschlusseinheit nach den Fig. 1, 2 und 5 unter Weglassung einzelner Bauteile,
- Fig. 7: in einer schematischen Perspektivdarstellung eine weitere Ausführungsform einer erfindungsgemäßen Anschlusseinheit und
- Fig. 8: in einer schematischen Perspektivdarstellung eine weitere Ausführungsform einer erfindungsgemäßen Anschlusseinheit.

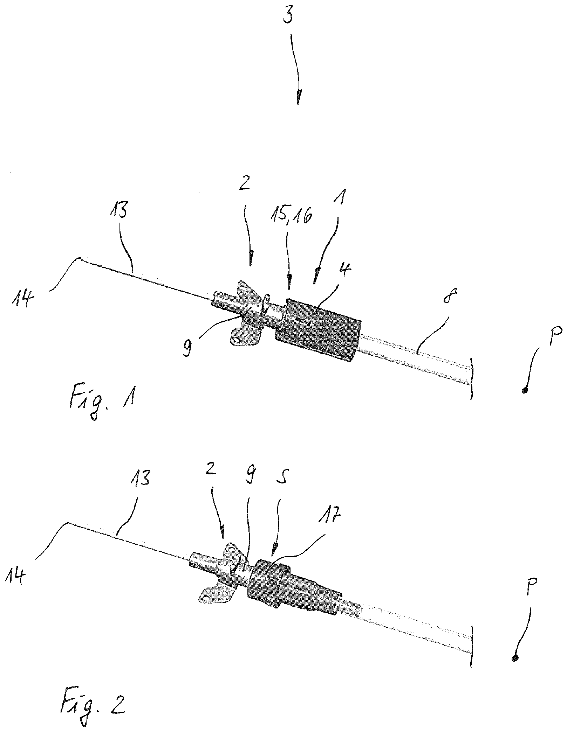

Gemäß Fig. 1 ist ein Verbindungssystem für ein medizinisches Fluidleitungssystem in Form einer Infusionsanordnung 3 zur periphervenösen Verabreichung einer medizinischen Flüssigkeit vorgesehen. Das Verbindungssystem weist eine Verbindungseinheit 1 und eine Anschlusseinheit 2 auf.

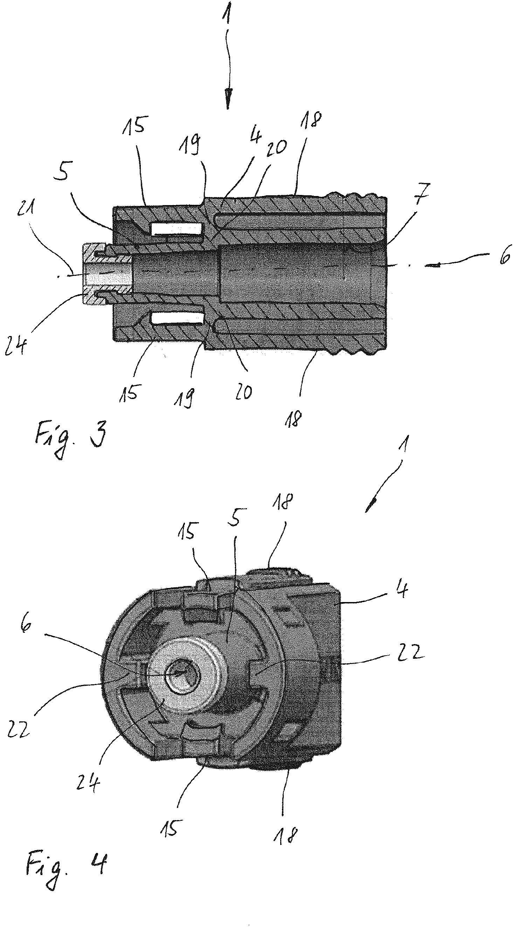

Die Verbindungseinheit 1 ist vorliegend in Form eines Infusionsschlauchverbinders ausgebildet, wobei die erfindungsgemäße Lösung nicht auf eine derartige Ausgestaltung der Verbindungseinheit beschränkt ist. Die Verbindungseinheit 1 weist einen Verbindungskörper 4 auf, der als Kunststoffspritzgussbauteil einstückig ausgeführt ist. Der Verbindungskörper 4 weist einen - in Bezug auf einen gedachten Referenzpunkt P - distal angeordneten Luer-Außenkegel 5 und koaxial zu den Luer-Außenkegel 5 erstreckten Durchgangskanal 6 auf (Fig. 3). Weiter weist der Verbindungskörper 4 einen proximal angeordneten Befestigungsabschnitt 7, der in Form einer radialen Aufweitung des Durchgangskanals 6 gebildet ist, auf. Der Befestigungsabschnitt 7 ist zur Verbindung mit einem Infusionsschlauch 8 vorgesehen. Zum Zwecke der Befestigung kann der Infusionsschlauch 8, wie anhand Fig. 1 ersichtlich, in den Befestigungsabschnitt 7 eingesteckt und/oder eingeklebt werden.

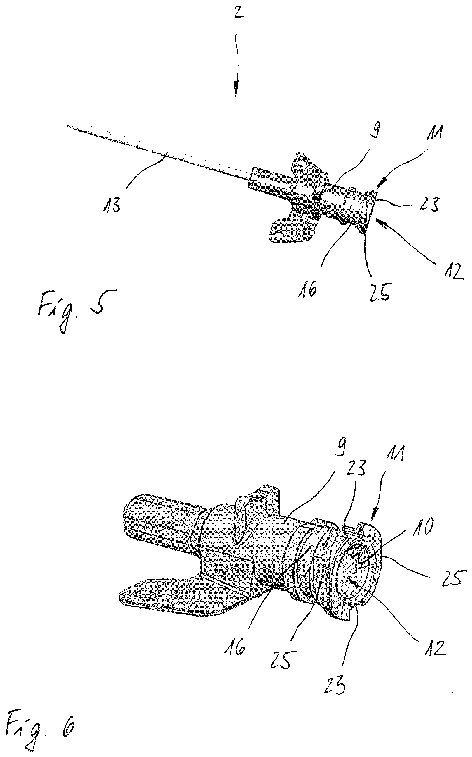

Die Anschlusseinheit 2 ist vorliegend in Form eines Venenverweilkatheters ausgebildet, wobei die erfindungsgemäße Lösung nicht auf eine derartige Ausgestaltung der Anschlusseinheit 2 beschränkt ist. Die Anschlusseinheit 2 weist einen Anschlusskörper 9 in Form eines Katheteransatzes auf. Der Katheteransatz 9 weist einen - in Bezug auf den Referenzpunkt P - proximal angeordneten Luer-Innenkegel 10, ein koaxial zu dem Luer-Innenkegel 10 sowie proximal angeordnetes Außengewinde 11 und ein koaxial zu dem Luer-Innenkegel 10 erstrecktes Lumen 12 auf (Fig. 6). An dem Katheteransatz 9 ist eine Katheternadel 13 angeordnet und fluidleitend mit dem Lumen 12 verbunden. In dem anhand Fig. 1 ersichtlichen Zustand ist der Luer-Außenkegel 5 der Verbindungseinheit 1 lösbar fluiddicht mit dem Luer-Innenkegel 10 des Katheteransatzes 9 verbunden. Eine solche Luer-Kegel-Verbindung ist im Bereich der Medizintechnik allgemein bekannt, wobei insbesondere die geometrische Ausgestaltung der Luer-Kegel 5, 10 üblicherweise einer Normung unterliegt. Mittels der Luer-Kegel-Verbindung wird eine fluidleitende Verbindung ausgehend von dem Infusionsschlauch 8 über den Durchgangskanal 6 in das Lumen 12 bis zu einer Austrittsöffnung 14 der Katheternadel 13 hergestellt.

Weiter sieht das Verbindungssystem gemäß Fig. 1 eine lösbare Rastverbindung 15, 16 zwischen wenigstens einem an dem Verbindungskörper 4 angeordneten Rastelement 15 (Fig. 3, 4) und wenigstens einem an dem Katheteransatz 9 angeordneten komplementären Gegenrastelement 16 (Fig. 5, 6) vor. Die Rastverbindung 15, 16 ist derart gestaltet, dass einem unbeabsichtigten Lösen der Verbindung der Luer-Kegel 5, 10 entgegengewirkt ist. Zudem sind das Gegenrastelement 16 und das Außengewinde 11 derart gestaltet und angeordnet, dass der Venenverweilkatheter 2, genauer: der Katheteransatz 9, in einem von der Verbindungseinheit 1 getrennten Zustand mittels einer Schraubverbindung S zwischen dem Außengewinde 11 und einem Innengewinde eines nach dem Stand der Technik ausgestalteten Luer-Lock-Ansatzes 17 verbindbar ist. Ein solcher Verbindungszustand des Venenverweilkatheters 2 ist anhand Fig. 2 ersichtlich. Demzufolge ermöglicht die noch näher zu erläuternde Ausgestaltung des Gegenrastelements 16 und des Außengewindes 11 wahlweise eine Verbindbarkeit des Katheteransatzes 9 mit einer Verbindungseinheit 1 nach den Fig. 1, 3 und 4 oder einem standardmäßigen Luer-Lock-Ansatz 17, wie er im Bereich der Medizintechnik allgemein bekannt ist.

Wie weiter anhand der Fig. 3 und 4 ersichtlich ist, weist die Verbindungseinheit 1 einen Handhabungsabschnitt 18 auf, der dem Rastelement 15 zugeordnet ist. Der Handhabungsabschnitt ist in Form eines axial erstreckten, leistenförmigen Betätigungshebels 18 ausgebildet. Das Rastelement 15 ist in Form eines axial und in distaler Richtung von dem Betätigungshebel 18 erstreckten Rasthaken 15 ausgebildet. Der Rasthaken 15 und der Betätigungshebel 18 sind stoffschlüssig miteinander verbunden und mittels eines Gelenkabschnitts 19 elastisch verschwenkbar an einer Innenwandung 20 des Verbindungskörpers 4 abgestützt. Der Gelenkabschnitt 19 ist in Form eines im Wesentlichen radial erstreckten Wandabschnitts des Verbindungskörpers 4 ausgebildet. Ausgehend von dem Gelenkabschnitt 19 ist der Betätigungshebel 18 in proximaler Richtung und der Rasthaken 15 in distaler Richtung erstreckt. In der anhand der Fig. 3 und 4 ersichtlichen Konfiguration nimmt der Rasthaken 15 eine Rastposition ein, in welcher der Rasthaken 15 in einem mit dem Katheteransatz 9 verbundenen Zustand der Verbindungseinheit 1 formschlüssig an dem Gegenrastelement 16 verrastet ist. Ausgehend von dieser Position ist der Rasthaken 15 mittels einer - in Bezug auf eine Mittellängsachse 21 der Verbindungseinheit 1 - radial nach innen gerichteten manuellen Druckbetätigung des Betätigungshebels 18 elastisch radial nach außen in eine Freigabeposition verlagerbar, in der der Rasthaken 15 das Gegenrastelement 16 freigibt. In der Freigabeposition sind die Luer-Kegel 5, 10 durch ein manuelles Abziehen der Verbindungseinheit 1 von dem Venenverweilkatheter 2 in Axialrichtung voneinander lösbar.

Wie weiter anhand der Fig. 3 und 4 ersichtlich ist, weist der Verbindungskörper 4 zwei spiegelsymmetrisch zueinander ausgebildete Rastelemente 15 auf. Jedem der Rastelemente 15 ist ein Handhabungsabschnitt 18 und ein Gelenkabschnitt 19 zugeordnet. Die Rastelemente 15 sind zusammen mit dem jeweils zugeordneten Handhabungsabschnitt 18 und dem jeweiligen Gelenkabschnitt 19 in Umfangsrichtung um 180° versetzt zueinander angeordnet und ansonsten hinsichtlich ihrer konstruktiven Ausgestaltung und Funktionsweise einander entsprechend. Weiter weist der Verbindungskörper 4 zwei in Umfangsrichtung um 180° versetzt zueinander angeordnete Führungsstege 22 auf. Die Führungsstege 22 sind zur axialen Führung an jeweils einer komplementären Führungsnut 23 des Katheteransatzes 9 vorgesehen. Die Führungsstege 22 sind in axialer Richtung längserstreckt und in Umfangsrichtung jeweils um 90° versetzt gegenüber den Rastelementen 15 angeordnet. Um eine verbesserte Abdichtung des Luer-Außenkegels 5 gegenüber dem Luer-Innenkegel 10 zu erreichen, ist zudem ein Dichtelement 24 vorgesehen. Das Dichtelement 24 weist gummielastische Eigenschaften auf und ist stirnendseitig an den Luer-Außenkonus 5 angespritzt.

Wie weiter anhand der Fig. 5 und 6 ersichtlich ist, ist das Gegenrastelement in Form einer in Umfangsrichtung erstreckten Radialnut 16 an dem Katheteransatz 9 ausgebildet. Die Radialnut 16 grenzt an einen distalen Stirnendbereich des Außengewindes 11 an. Gegenüber dem Außengewinde 11 ist die Radialnut 16 in radialer Richtung zurückversetzt, so dass das Außengewinde 11 die Radialnut 16 in radialer Richtung überragt. Um anstelle der anhand Fig. 1 ersichtlichen Verbindung der Anschlusseinheit 2 mit der Verbindungseinheit 1 eine Verbindbarkeit mit dem standardmäßigen Luer-Lock-Ansatz 17 zu gewährleisten, ist das Außengewinde 11 - im Vergleich zu einer standardmäßigen Luer-Lock-Gewindegeometrie - spezifisch ausgestaltet. Zum einen ist das Außengewinde 11 in Axialrichtung von den zwei Führungsnuten 23, die zur Führung der Führungsstege 22 der Verbindungseinheit 1 vorgesehen sind, durchsetzt. Zum anderen weist das Außengewinde abgeflachte Gewindeabschnitte 25 auf. Die abgeflachten Gewindeabschnitte 25 sind in Umfangsrichtung um 180° versetzt zueinander und jeweils um 90° versetzt zu den Führungsnuten 23 angeordnet. Gegenüber dem Außendurchmesser des Außengewindes 23 sind die abgeflachten Gewindeabschnitte 25 radial zurückgesetzt.

Zum lösbaren Verbinden der Verbindungseinheit 1 mit der Anschlusseinheit 2 werden die Einheiten 1, 2 axial fluchtend und mit den Luer-Kegeln 5, 10 gegeneinanderweisend ausgerichtet. Die Führungsstege 22 der Verbindungseinheit 1 werden stirnendseitig an die jeweilige Führungsnut 23 der Anschlusseinheit 2 angesetzt. Weiter werden die Verbindungseinheit 1 und die Anschlusseinheit 2 in axialer Richtung gegeneinander bewegt, wobei der Luer-Außenkegel 5 in den Luer-Innenkegel 10 eindringt und die Rastelemente 15 über die abgeflachten Gewindeabschnitte 25 hinweggeführt werden. Um ein vereinfachtes Hinwegführen der Rastelemente 15 über die abgeflachten Gewindeabschnitte 25 zu ermöglichen, können die Handhabungsabschnitte 18 bedarfsweise radial nach innen gedrückt werden, was jedoch nicht zwingend erforderlich ist. Nach einem vollständigen Eindringen des Luer-Außenkegels 5 in den Luer-Innenkegel 10 wird eine fluiddichte Verbindung erreicht, wobei die Rastelemente 15 in der Radialnut 16 verrasten.

Zum Lösen der Luer-Kegel-Verbindung erfolgt der vorbeschriebene Bewegungsablauf im Wesentlichen in umgekehrter Abfolge, wobei zunächst die Handhabungsabschnitte 18 in radialer Richtung nach innen gedrückt und derart die formschlüssige Verbindung zwischen den Rastelementen 15 und der Radialnut 16 aufgehoben wird.

Ein Verbinden und/oder Lösen der Anschlusseinheit 2 mit bzw. von dem Luer-Lock-Ansatz 17 erfolgt entsprechend zu einer üblichen Luer-Lock-Verbindung, wie sie im Bereich der Medizintechnik allgemein bekannt ist.

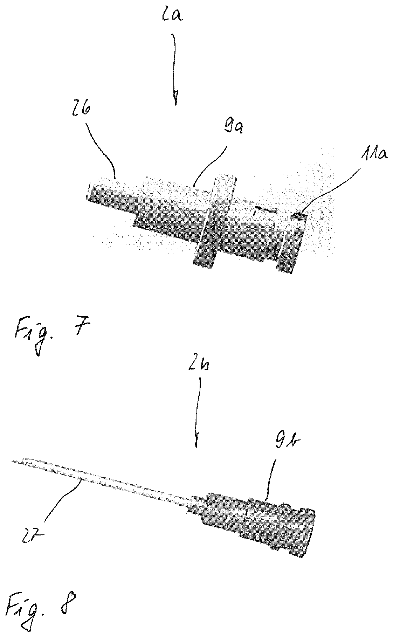

Weitere Ausführungsformen erfindungsgemäßer Anschlusseinheiten 2a, 2b sind anhand der Fig. 7 bzw. 8 ersichtlich. Die Ausführungsformen nach den Fig. 7 und 8 entsprechen im Wesentlichen der zuvor anhand der Fig. 1, 2 sowie 5 und 6 beschriebenen Ausführungsform. Zur Vermeidung von Wiederholungen wird daher auf die Offenbarung zu der Ausführungsform nach den letztgenannten Figuren verwiesen. Nachfolgend wird lediglich auf die wesentlichen Unterschiede der Anschlusseinheiten 2a, 2b gegenüber der Anschlusseinheit 2 eingegangen. Identische Bauteile und Abschnitte sind insoweit mit identischen Bezugszeichen versehen und zur Vermeidung von Wiederholungen nicht bei jeder Ausführungsform gesondert erläutert. Funktionsgleiche Bauteile und Abschnitte, die jedoch in ihrer konstruktiven Ausführung unterschiedlich sind, sind mit gleichen Bezugszeichenziffern unter Hinzufügung von Kleinbuchstaben bezeichnet.

Die Anschlusseinheit 2a ist in Form eines Schlauchverbinders gestaltet. Der Schlauchverbinder 2a sieht einen Anschlusskörper 9a vor, an dessen distalem Ende ein Schlauchansatz 26 angeordnet ist. Weiter unterscheidet sich der Schlauchverbinder 2a dahingehend von der Anschlusseinheit 2, dass anstelle eines durchgängigen Gewindegangs lediglich ein Gewindenocken 11a vorgesehen ist. Insoweit kann auf eine Abflachung 25 nach Fig. 6 verzichtet werden.

Die Anschlusseinheit 2b ist in Form einer Einwegkanüle gestaltet. Die Einwegkanüle 2b weist einen Nadelansatz 9b auf. Distal an dem Nadelansatz 9b ist eine hypodermische Nadel 27 angeordnet. Der zur Verbindung mit der Verbindungseinheit 1 oder - stattdessen - dem Luer-Lock-Ansatz 17 vorgesehene proximale Bereich des Nadelansatzes 9b ist im Übrigen im Wesentlichen übereinstimmend zu der Ausführungsform nach Fig. 7 ausgestaltet.

## Patentansprüche

1. Verbindungssystem für ein medizinisches Fluidleitungssystem (3) aufweisend eine Verbindungseinheit (1) mit einem Verbindungskörper (4), der einen distal angeordneten Luer-Außenkegel (5) und einen koaxial zu dem Luer-Außenkegel (5) erstreckten Durchgangskanal (6) aufweist, und eine Anschlusseinheit (2) mit einem Anschlusskörper (9), der einen proximal angeordneten Luer-Innenkegel (10), der lösbar fluiddicht mit dem Luer-Außenkegel (5) der Verbindungseinheit (1) verbunden ist, ein koaxial zu dem Luer-Innenkegel (10) sowie proximal angeordnetes Außengewinde (11), das zur Verbindung mit einem Innengewinde eines Luer-Lock-Ansatzes (17) geeignet ist, und ein koaxial zu dem Luer-Innenkegel (10) erstrecktes und mittels der Verbindung der Luer-Kegel (5, 10) lösbar fluidleitend mit dem Durchgangskanal (6) verbundenes Lumen (12) aufweist, **dadurch gekennzeichnet, dass** eine lösbare Rastverbindung (15, 16) zwischen wenigstens einem an dem Verbindungskörper (4) angeordneten Rastelement (15) und wenigstens einem an dem Anschlusskörper (9) angeordneten komplementären Gegenrastelement (16) vorgesehen ist, wobei die Rastverbindung (15, 16) derart gestaltet ist, dass einem unbeabsichtigten Lösen der Verbindung der Luer-Kegel (5, 10) entgegengewirkt ist, und wobei das Gegenrastelement (16) und das Außengewinde (11) derart gestaltet und angeordnet sind, dass die Anschlusseinheit (2) - in einem von der Verbindungseinheit (1) getrennten Zustand - mittels einer Schraubverbindung (S) zwischen dem Außengewinde (11) und dem Innengewinde des Luer-Lock-Ansatzes (17) verbindbar ist, wobei das Gegenrastelement (16) an einen distalen Stirnendbereich des Außengewindes (11) angrenzt und in radialer Richtung gegenüber einem Durchmesser des Außengewindes (11) zurückversetzt ist.

2. Verbindungseinheit (1) für ein Verbindungssystem nach Anspruch 1 und/oder zur lösbaren Wirkverbindung mit einer Anschlusseinheit (2 bis 2b) nach einem der Ansprüchen 9 bis 12 mit einem Verbindungskörper (4), der einen distal angeordneten Luer-Außenkegel (5) und einen koaxial zu dem Luer-Außenkegel (5) erstreckten Durchgangskanal (6) aufweist, **dadurch gekennzeichnet, dass** der Verbindungskörper (4) ein Rastelement (15) und einen dem Rastelement (15) zugeordneten Handhabungsabschnitt (18) aufweist, wobei das Rastelement (15) und der Handhabungsabschnitt (18) derart angeordnet und/oder gestaltet sind, dass das Rastelement (15) mittels einer manuellen Handhabung des Handhabungsabschnitts (18) - in Bezug auf einen mit der Anschlusseinheit (2 bis 2b) verbundenen Zustand - relativ zu dem Luer-Außenkegel (5) von einer Rastposition, in der das Rastelement (15) an einem Gegenrastelement (16) der Anschlusseinheit (2 bis 2b) verrastet ist, in eine Freigabeposition, in der das Rastelement (15) das Gegenrastelement (16) freigibt, verlagerbar ist.

3. Verbindungseinheit (1) nach Anspruch 2, **dadurch gekennzeichnet, dass** das Rastelement (15) mittels einer - in Bezug auf eine Mittellängsachse (21) der Verbindungseinheit (1) - radial nach innen gerichteten elastischen Verlagerung des Handhabungsabschnitts (18) radial nach außen elastisch in die Freigabeposition verlagerbar ist.

4. Verbindungseinheit (1) nach Anspruch 2 oder 3, **dadurch gekennzeichnet, dass** der Verbindungskörper (4) einen biegeelastischen Gelenkabschnitt (19) aufweist, an dem das Rastelement (15) in distaler Richtung und der Handhabungsabschnitt (18) in proximaler Richtung erstreckt und in einer Mittellängsebene der Verbindungseinheit (1) verschwenkbar angelenkt sind.

5. Verbindungseinheit (1) nach Anspruch 4, **dadurch gekennzeichnet, dass** mehrere, vorzugsweise zwei, in Umfangsrichtung gleichmäßig versetzt zueinander angeordnete Rastelemente (15) mit jeweils einem zugeordneten Handhabungsabschnitt (18) sowie einem Gelenkabschnitt (19) vorgesehen sind.

6. Verbindungseinheit (1) nach einem der Ansprüche 2 bis 5, **dadurch gekennzeichnet, dass** der Verbindungskörper (4) wenigstens einen in axialer Richtung erstreckten Führungssteg (22) aufweist, der zur axialen Führung in einer komplementären Führungsnut (23) der Anschlusseinheit (2 bis 2b) vorgesehen ist.

7. Verbindungseinheit (1) nach Anspruch 6, **dadurch gekennzeichnet, dass** mehrere, vorzugsweise zwei, in Umfangsrichtung gleichmäßig versetzt zueinander angeordnete Führungsstege (22) vorgesehen sind.

8. Verbindungseinheit (1) nach einem der Ansprüche 2 bis 7, **dadurch gekennzeichnet, dass** ein stirnendseitig an den Luer-Außenkonus (5) angespritztes Dichtelement (24) vorgesehen ist.

9. Anschlusseinheit (2 bis 2b) zur lösbaren Wirkverbindung mit einem Luer-Lock-Ansatz (17) und/oder einer Verbindungseinheit (1) nach einem der Ansprüche 2 bis 8 mit einem Anschlusskörper (9 bis 9b), der einen proximal angeordneten Luer-Innenkegel (10), der lösbar fluiddicht mit dem Luer-Außenkegel (5) der Verbindungseinheit (1) verbindbar ist, ein koaxial zu einem Luer-Innenkegel (10) und proximal angeordnetes Außengewinde (11, 11a) und ein koaxial zu dem Luer-Innenkegel (10) erstrecktes Lumen (12) aufweist, **dadurch gekennzeichnet, dass** der Anschlusskörper (9 bis 9b) ein Gegenrastelement (16) zur lösbaren Rastverbindung mit einem Rastelement (15) der Verbindungseinheit (1) aufweist, und wobei das Gegenrastelement (16) und das Außengewinde (11, 11a) derart angeordnet und gestaltet sind, dass die Anschlusseinheit (2 bis 2b) mit der Verbindungseinheit (1) mittels einer Rastverbindung (15, 16) zwischen dem Gegenrastelement (16) und dem Rastelement (15) und - stattdessen - mittels einer Schraubverbindung (S) zwischen dem Außengewinde (11, 11a) und einem Innengewinde des Luer-Lock-Ansatzes (17) verbindbar ist wobei das Gegenrastelement (16) an einen distalen Stirnendbereich des Außengewindes (11, 11a) angrenzt und in radialer Richtung gegenüber einem Durchmesser des Außengewindes (11, 11a) zurückversetzt ist.

10. Anschlusseinheit (2 bis 2b) nach Anspruch 9, **dadurch gekennzeichnet, dass** das Gegenrastelement in Form einer in Umfangsrichtung, vorzugsweise vollständig umlaufend, erstreckten Radialnut (16) an dem Anschlusskörper (9 bis 9b) ausgebildet ist.

11. Anschlusseinheit (2 bis 2b) nach Anspruch 9 oder 10, **dadurch gekennzeichnet, dass** das Außengewinde (11, 11a) in Axialrichtung von wenigstens einer Führungsnut (23), die zur Führung eines Führungsstegs (22) der Verbindungseinheit (1) vorgesehen ist, durchsetzt ist.

12. Anschlusseinheit (2) nach einem der Ansprüche 9 bis 11, **dadurch gekennzeichnet, dass** das Außengewinde (11) wenigstens einen gegenüber einem Außendurchmesser des Außengewindes (11) radial abgeflachten Gewindeabschnitt (25) aufweist, der - in einem mit der Verbindungseinheit (1) verbundenen Zustand der Anschlusseinheit (2) - von dem Rastelement (15) hintergriffen ist.

## Claims

1. Connection system for a medical fluid line system (3) having a connection unit (1) with a connection body (4), which has a distally arranged Luer outer cone (5) and a through-channel (6) extending coaxially with respect to the Luer outer cone (5), and an attachment unit (2) with an attachment body (9) which has a proximally arranged Luer inner cone (10), connected releasably and in a fluid-tight manner to the Luer outer cone (5) of the connection unit (1), a proximally arranged external thread (11) which is coaxial with respect to the Luer inner cone (10) and is suitable for connection to an internal thread of a Luer lock connector (17), and a lumen (12) which extends coaxially with respect to the Luer inner cone (10) and which is releasably connected in a fluid-conducting manner to the through-channel (6) by means of the connection of the Luer cones (5, 10), **characterized in that** a releasable locking connection (15, 16) is provided between at least one locking element (15) arranged on the connection body (4) and at least one complementary counter-locking element (16) arranged on the attachment body (9), wherein the locking connection (15, 16) is designed in such a way as to counteract an unintentional release of the connection of the Luer cones (5, 10), and wherein the counter-locking element (16) and the external thread (11) are designed and arranged in such a way that the attachment unit (2), in a state when separate from the connection unit (1), is connectable by means of a screw connection (S) between the external thread (11) and the internal thread of the Luer lock connector (17), wherein the counter-locking element (16) is adjacent to a distal end region of the external thread (11) and is radially set back relative to a diameter of the external thread (11) .

2. Connection unit (1) for a connection system according to Claim 1 and/or for releasable operative connection to an attachment unit (2 to 2b) according to any one of Claims 9 to 12, with a connection body (4) which has a distally arranged Luer outer cone (5) and a through-channel (6) extending coaxially with respect to the Luer outer cone (5), **characterized in that** that the connection body (4) has a locking element (15) and a handling section (18) associated with the locking element (15), wherein the locking element (15) and the handling section (18) are arranged and/or designed in such a way that, by means of a manual handling of the handling section (18) in relation to a state when connected to the attachment unit (2 to 2b), the locking element (15) is displaceable relative to the Luer outer cone (5) from a locking position, in which the locking element (15) is latched onto a counter-locking element (16) of the attachment unit (2 to 2b), to a release position, in which the locking element (15) releases the counter-locking element (16).

3. Connection unit (1) according to Claim 2, **characterized in that** the locking element (15) is displaceable radially outwardly and elastically to the release position by means of a radially inwardly directed elastic displacement of the handling section (18) relative to a central longitudinal axis (21) of the connection unit (1).

4. Connection unit (1) according to Claim 2 or 3, **characterized in that** the connection body (4) has a flexible joint section (19), on which the locking element (15) extends in the distal direction and the handling section (18) extends in the proximal direction and on which the locking element and handling section are pivotably hinged in a central longitudinal plane of the connection unit (1).

5. Connection unit (1) according to Claim 4, **characterized in that** several, preferably two, locking elements (15) are provided, these being uniformly offset with respect to one another in the circumferential direction and each having an associated handling section (18) and a joint section (19).

6. Connection unit (1) according to any one of Claims 2 to 5, **characterized in that** the connection body (4) has at least one guide web (22) which extends in the axial direction and which is provided for axial guidance in a complementary guide groove (23) of the attachment unit (2 to 2b) .

7. Connection unit (1) according to Claim 6, **characterized in that** several, preferably two, guide webs (22) are provided, these being uniformly offset with respect to one another in the circumferential direction.

8. Connection unit (1) according to any one of Claims 2 to 7, **characterized in that** a sealing element (24) is provided that is injected at the end face onto the Luer outer cone (5).

9. Attachment unit (2 to 2b) for releasable operative connection to a Luer lock connector (17) and/or to a connection unit (1) according to any one of Claims 2 to 8, with an attachment body (9 to 9b) which has a proximally arranged Luer inner cone (10), connectable releasably and in a fluid-tight manner to the Luer outer cone (5) of the connection unit (1), a proximally arranged external thread (11, 11a) coaxial with respect to a Luer inner cone (10), and a lumen (12) extending coaxially with respect to the Luer inner cone (10), **characterized in that** the attachment body (9 to 9b) has a counter-locking element (16) for releasable locking connection to a locking element (15) of the connection unit (1), and wherein the counter-locking element (16) and the external thread (11, 11a) are arranged and designed in such a way that the attachment unit (2 to 2b) is connectable to the connection unit (1) by means of a locking connection (15, 16) between the counter-locking element (16) and the locking element (15) and - instead - by means of a screw connection (S) between the external thread (11, 11a) and an internal thread of the Luer lock connector (17), wherein the counter-locking element (16) is adjacent to a distal end region of the external thread (11, 11a) and is radially set back relative to a diameter of the external thread (11, 11a).

10. Attachment unit (2 to 2b) according to Claim 9, **characterized in that** the counter-locking element is designed in the form of a radial groove (16) extending in the circumferential direction, preferably completely about the circumference, on the attachment body (9 to 9b) .

11. Attachment unit (2 to 2b) according to Claim 9 or 10, **characterized in that** the external thread (11, 11a) is traversed in the axial direction by at least one guide groove (23), which is provided for guiding a guide web (22) of the connection unit (1).

12. Attachment unit (2) according to any one of Claims 9 to 11, **characterized in that** the external thread (11) has at least one thread section (25) radially flattened with respect to an external diameter of the external thread (11), which thread section, in a state with the attachment unit (2) connected to the connection unit (1), is engaged from behind by the locking element (15).

## Revendications

1. Système de raccordement pour un système (3) de conduites de fluide médical, présentant une unité de connexion (1) ayant un corps de connexion (4) qui présente un cône extérieur Luer (5) agencé de manière distale et un conduit de passage (6) s'étendant de manière coaxiale au cône extérieur Luer (5), et une unité de raccordement (2) ayant un corps de raccordement (9), qui présente un cône intérieur Luer (10) agencé de manière proximale, qui est relié au cône extérieur Luer (5) de l'unité de connexion (1) de manière amovible et étanche aux fluides, un filet extérieur (11) agencé coaxialement au cône intérieur Luer (10) ainsi que proximalement, qui est adapté pour un raccordement avec un filetage intérieur d'un embout Luer-Lock (17), et une lumière (12) s'étendant coaxialement au cône intérieur Luer (10) et reliée de manière détachable et conductrice de fluide au conduit de passage (6) au moyen du raccordement des cônes Luer (5, 10), **caractérisé en ce qu'**un raccordement par encliquetage détachable (15, 16) est prévu entre au moins un élément d'encliquetage (15) agencé sur le corps de connexion (4) et au moins un contre-élément d'encliquetage (16) complémentaire agencé sur le corps de raccordement (9), le raccordement par encliquetage (15, 16) étant conçu de telle sorte qu'il s'oppose à une libération involontaire du raccordement des cônes Luer (5, 10), et le contre-élément d'encliquetage (16) et le filet extérieur (11) étant conçus et agencés de telle sorte que l'unité de raccordement (2) - dans un état séparé de l'unité de connexion (1) - est apte à être reliée au moyen d'un raccordement par vissage (S) entre le filet extérieur (11) et le filetage intérieur de l'embout Luer-Lock (17), le contre-élément d'encliquetage (16) étant adjacent à une zone d'extrémité frontale distale du filet extérieur (11) et étant décalé en arrière dans la direction radiale par rapport à un diamètre du filet extérieur (11).

2. Unité de connexion (1) pour un système de raccordement selon la revendication 1 et/ou pour un raccordement actif amovible avec une unité de raccordement (2 à 2b) selon l'une des revendications 9 à 12, ayant un corps de connexion (4) qui présente un cône extérieur Luer (5) agencé de manière distale et un conduit de passage (6) s'étendant coaxialement au cône extérieur Luer (5), **caractérisée en ce que** le corps de connexion (4) présente un élément d'encliquetage (15) et une portion de manipulation (18) associée à l'élément d'encliquetage (15), l'élément d'encliquetage (15) et la portion de manipulation (18) étant agencés et/ou conçus de telle sorte que l'élément d'encliquetage (15) soit apte à être déplacé, au moyen d'une manipulation manuelle de la portion de manipulation (18) - par rapport à un état raccordé à l'unité de raccordement (2 à 2b) - relativement au cône extérieur Luer (5) d'une position d'encliquetage, dans laquelle l'élément d'encliquetage (15) est encliqueté sur un élément d'encliquetage complémentaire (16) de l'unité de raccordement (2 à 2b), jusqu'à une position de libération dans laquelle l'élément d'encliquetage (15) libère le contre-élément d'encliquetage (16).

3. Unité de connexion (1) selon la revendication 2, **caractérisée en ce que** l'élément d'encliquetage (15) est apte à être déplacé élastiquement dans la position de libération, radialement vers l'extérieur, au moyen d'un déplacement élastique de la portion de manipulation (18), dirigé radialement vers l'intérieur - par rapport à un axe longitudinal central (21) de l'unité de connexion (1) .

4. Unité de connexion (1) selon la revendication 2 ou la revendication 3, **caractérisée en ce que** le corps de connexion (4) présente une section d'articulation (19) élastique en flexion, sur laquelle l'élément d'encliquetage (15) s'étend en direction distale et la portion de manipulation (18) s'étend en direction proximale et ils sont articulés de manière pivotante dans un plan longitudinal médian de l'unité de connexion (1).

5. Unité de connexion (1) selon la revendication 4, **caractérisée en ce qu'**il est prévu plusieurs, de préférence deux, éléments d'encliquetage (15) agencés de manière régulièrement décalée les uns par rapport aux autres dans la direction circonférentielle, avec respectivement une portion de manipulation (18) associée ainsi qu'une portion d'articulation (19).

6. Unité de connexion (1) selon l'une des revendications 2 à 5, **caractérisée en ce que** le corps de connexion (4) présente au moins une nervure de guidage (22) s'étendant dans la direction axiale, qui est prévue pour le guidage axial dans une rainure de guidage (23) complémentaire de l'unité de raccordement (2 à 2b).

7. Unité de connexion (1) selon la revendication 6, **caractérisée en ce qu'**il est prévu plusieurs, de préférence deux, nervures de guidage (22) agencées de manière régulièrement décalée les unes par rapport aux autres dans la direction circonférentielle.

8. Unité de connexion (1) selon l'une des revendications 2 à 7, **caractérisée en ce qu'**il est prévu un élément d'étanchéité (24) moulé par injection du côté frontal sur le cône extérieur Luer (5).

9. Unité de raccordement (2 à 2b) pour un raccordement actif amovible avec un embout Luer-Lock (17) et/ou une unité de connexion (1) selon l'une des revendications 2 à 8, avec un corps de raccordement (9 à 9b) qui présente un cône intérieur Luer (10) agencé de manière proximale, qui est apte à être relié de manière amovible et étanche aux fluides au cône extérieur Luer (5) de l'unité de connexion (1), présente un filet extérieur (11, 11a) agencé coaxialement à un cône intérieur Luer (10) et proximalement et une lumière (12) s'étendant coaxialement au cône intérieur Luer (10), **caractérisé en ce que** le corps de raccordement (9 à 9b) présente un contre-élément d'encliquetage (16) pour le raccordement par encliquetage détachable avec un élément d'encliquetage (15) de l'unité de connexion (1), et dans lequel le contre-élément d'encliquetage (16) et le filet extérieur (11, 11a) sont agencés et conçus de telle sorte que l'unité de raccordement (2 à 2b) est apte à être reliée à l'unité de connexion (1) au moyen d'un raccordement par encliquetage (15, 16) entre le contre-élément d'encliquetage (16) et l'élément d'encliquetage (15) et - à la place - au moyen d'un raccordement par vissage (S) entre le filet extérieur (11, 11a) et un filetage intérieur de l'embout Luer-Lock (17), le contre-élément d'encliquetage (16) étant adjacent à une zone d'extrémité frontale distale du filet extérieur (11, 11a) et étant décalé en arrière dans la direction radiale par rapport à un diamètre du filet extérieur (11, 11a).

10. Unité de raccordement (2 à 2b) selon la revendication 9, **caractérisée en ce que** le contre-élément d'encliquetage est réalisé sur le corps de raccordement (9 à 9b) sous la forme d'une rainure radiale (16) s'étendant dans la direction circonférentielle, de préférence sur tout le pourtour.

11. Unité de raccordement (2 à 2b) selon la revendication 9 ou la revendication 10, **caractérisée en ce que** le filet extérieur (11, 11a) est traversé dans la direction axiale par au moins une rainure de guidage (23) qui est prévue pour guider une nervure de guidage (22) de l'unité de connexion (1).

12. Unité de raccordement (2) selon l'une des revendications 9 à 11, **caractérisée en ce que** le filet extérieur (11) présente au moins une section filetée (25) aplatie radialement par rapport à un diamètre extérieur du filet extérieur (11), qui - dans un état de connexion de l'unité de raccordement (2) à l'unité de connexion (1) - est engagée par l'arrière par l'élément d'encliquetage (15).
